(19) **Europäisches Patentamt**

**European Patent Office**

**Office européen des brevets**

(11) **EP 1 053 030 B1**

(12) **EUROPÄISCHE PATENTSCHRIFT**

(45) Veröffentlichungstag und Bekanntmachung des Hinweises auf die Patenterteilung:
**30.10.2002   Patentblatt 2002/44**

(51) Int Cl.$^7$: **A61L 15/44**, A61L 15/58

(21) Anmeldenummer: **99904855.6**

(86) Internationale Anmeldenummer:
**PCT/EP99/00738**

(22) Anmeldetag: **04.02.1999**

(87) Internationale Veröffentlichungsnummer:
**WO 99/039756 (12.08.1999 Gazette 1999/32)**

(54) **TRÄGERMATERIAL FÜR MEDIZINISCHE ZWECKE**

SUPPORTING MATERIAL FOR MEDICINAL PURPOSES

MATERIAL DE SUPPORT A USAGE MEDICAL

(84) Benannte Vertragsstaaten:
**AT DE ES FR GB IT NL SE**

(30) Priorität: **06.02.1998  DE 19804774**

(43) Veröffentlichungstag der Anmeldung:
**22.11.2000   Patentblatt 2000/47**

(73) Patentinhaber: **Beiersdorf Aktiengesellschaft 20245 Hamburg (DE)**

(72) Erfinder:
- **HIMMELSBACH, Peter D-21614 Buxtehude (DE)**
- **SINNEN, Marike D-25421 Pinneberg (DE)**

(56) Entgegenhaltungen:
| | |
|---|---|
| DE-A- 4 237 252 | DE-A- 4 442 092 |
| DE-A- 19 531 291 | DE-A- 19 631 422 |
| DE-C- 4 224 325 | US-A- 4 967 740 |
| US-A- 5 453 319 | |

Printed by Jouve, 75001 PARIS (FR)

**Beschreibung**

Die Erfindung betrifft ein Trägermaterial für medizinische Zwecke, vorzugsweise ein medizinisches Pflaster, welches zumindest einseitig vollflächig oder partiell mit einer dotierten Klebemasse beschichtet ist.

Als Trägermaterialien für medizinische Zwecke sind bereits zahlreiche Materialien auf Folien-, Gewebe-, Gewirke-, Vlies-, Gel- oder Schaumstoff-Basis bekannt und werden auch in der Praxis eingesetzt. Die Materialien, welche oft noch mit einer Selbstklebemasse beschichtet sind, müssen hautverträglich, in der Regel luft- und wasserdampfdurchlässig sowie gut anmodellierbar und anschmiegsam sein. Aufgrund dieser Anforderungen wird häufig ein möglichst dünner oder weicher Träger bevorzugt. Zur Handhabung und beim Gebrauch sind bei den Trägermaterialien aber auch eine ausreichende Festigkeit und gegebenenfalls begrenzte Dehnbarkeit gefordert. Weiterhin sollte das Trägermaterial auch nach dem Durchnässen eine ausreichende Festigkeit und geringe Dehnbarkeit aufweisen.

Dünne Träger, insbesondere solche aus Vliesen, sind gut luft- und wasserdampfdurchlässig. Für bestimmte Anwendungen ist jedoch ihre Festigkeit zu gering und ihre Dehnung zu hoch. Weiter reicht der Polsterungseffekt für diese günstigen Träger nicht aus.

Für spezielle Anwendungen, zum Beispiel Rheumapflaster sind unelastische Träger mit einer hohen Festigkeit in Beanspruchungsrichtung erforderlich. Dies wird erreicht, indem Gewebe- oder Gewirketräger, üblicherweise aus Baumwolle oder Viskose, eingesetzt werden. In der Regel sind solche Trägermaterialien mit entsprechend hohem Flächengewicht kostenintensiv. Eine hohe Anschmiegsamkeit und Anmodellierbarkeit ist nur durch Gewebe mit geringerer Festigkeit zu erreichen. Diese weisen im allgemeinen aber dann unter Beanspruchung eine zu geringe Polsterung auf, weiche für die Anwendung unerwünscht ist.

Generell können beim Durchnässen die aufgeführten Verbände an Festigkeit verlieren oder stärker dehnbar sein. Dieses ist ebenfalls für die Anwendung unerwünscht und wird bisher durch ein häufigeres Verbandswechseln, welches auf der anderen Seite kostenintensiv ist, kompensiert.

So beschreibt die AU 73555/74 beispielhaft ein mit Glasfäden verstärktes Trägermaterial für medikale Anwendungen auf Schaumbasis.

US 4,668,563 beschreibt ein mit Glasfaser verstärktes Material, welches jedoch elastisch ist. Alle genannten Trägermateriallen sind jedoch nicht durch Vemähungen verstärkt worden.

DE 44 42 092 und DE G 94 01 037 beschreiben Klebebänder auf Nähvliesbasis, welche teilweise auf der Trägerrückseite beschichtet sind. Solche Klebebänder finden dabei bevorzugt in der Kabelbandagierung Verwendung. Die in DE 44 42 092 vorgesehene lack-beschichtung macht das Klebeband unvereinbar mit einer medizinischen Anwendung.

Der DE 44 42 093 liegt auch die Verwendung eines Vlieses als Träger für ein Klebeband zugrunde, hier wird ein durch die Bildung von Maschen aus den Fasern des Vlieses verstärktes Querfaservlies beschrieben.

DE 44 42 507 offenbart ebenfalls ein Klebeband zur Kabeibandagierung, jedoch basiert es auf sogenannten Kunit- beziehungsweise Multikunitvliesen.

Eine Verwendung oder spezielle Eignung dieser Trägermaterialien für medizinische Zwecke ist jedoch nicht aus den genannten Schriften abzuleiten. Insbesondere sind in den Veröffentlichungen weder Hinweise auf eine ausreichende Hautverträglichkeit der Klebemassen, noch auf ein funktionell sicheres Verkleben auf der Haut oder eine vorteilhafte Luft- und Wasserdampfdurchlässigkeit gegeben. Weiter ist eine Dotierung mit aktiven Substanzen nicht erwähnt.

US 4,773,238 beschreibt ein längs übemähtes Faservlies, wobei die Nähnähte nicht mehr als 20 Gew.-% bezogen auf das Gewicht des gesamten Vlieses haben sollen. Erfindungsgemäß vorgeschlagen wird die Verwendung als Einlage für Filter in der Staubfilterung.

Aus US 4,967,740 werden allgemein Trägermaterialien zur Verwendung in der medizinischen Versorgung offenbart, die in einem 1-Schritt-Verfahren hergestellt werden. In diesem Verfahren wird der Träger gleichzeitig mit einem Elastomer und mit einer Releaselösung imprägniert. Das Material kann auf diese Weise ohne Trennpapier auf einer Rolle dargeboten werden. Eine Dotierung mit aktiven Substanzen nicht erwähnt.

In einer Aufzählung einer Vielzahl geeigneter Träger werden auch übernähte Vliese genannt, ohne dem Fachmann einen Hinweis zu geben, wie das Vlies konkret ausgeführt sein soll, um die an einen medizinischen Träger gestellten Anforderungen zu erfüllen.

Transdermale Therapeutische Systeme (TTS) sind Darreichungsformen von Medikamenten, die einen oder mehrere Arzneistoffe über einen definierten Zeitraum an ihrem Anwendungsort an die Haut abgeben. Es wird dabei zwischen systemisch und lokal wirksamen Darreichungsformen unterschieden.

Bei systemisch wirkenden Darreichungsformen gelangt der Wirkstoff durch Diffusion durch die Haut in den Blutkreislauf und kann im ganzen Körper wirken.

Lokal wirkende Darreichungsformen wirken dagegen nur an den applizierten Stellen. Der Wirkstoff verbleibt in der Haut beziehungsweise in den darunter liegenden Schichten.

Stark klebende Pflaster werden, üblicherweise vollflächig mit einer Kautschuk-Klebemasse beschichtet. Diese Klebemassen erlauben dann ein sicheres Verkleben zum Beispiel an kritischen Stellen wie Gelenken über die Dauer von mehreren Tagen. Das Verkleben derartiger Produkte auf der Haut zeigt jedoch nach dem Ablösen deutliche Hautirritationen und eine mechanische Beanspruchung der Haut. Die Verklebung läßt sich ohne Hilfsmittel nur unter Schmerzen lösen.

Weitere Klebemassen basieren auf Acrylatsystemen oder auf Blockcopolymeren.

Die zuvor genannten Klebemassen können druckempfindliche Selbstklebemassen sein, wobei die Massen für die Verarbeitung in einer Trägermatrix vorliegen können. Als Trägermatrix werden gängige organische oder anorganische Lösemittel oder Dispergiermittel verstanden.

Systeme ohne Trägermatrix werden als 100 %-Systeme bezeichnet und sind ebenfalls nicht unbekannt. Sie werden im elastischen oder thermoplastischen Zustand verarbeitet. Eine gängige Verarbeitungsweise ist die Schmelze. Auch solche Haftschmelzklebemassen sind im Stande der Technik bereits vorbeschrieben. Sie basieren auf natürlichen oder synthetischen Kautschuken und/oder anderen synthetischen Polymeren.

Vorteilhaft an den 100 %-Systemen ist, daß bei ihnen verfahrenstechnisch ein Entfernen der Trägermatrix, d.h. der Hilfsmittel, vermieden wird, wodurch sich die Verarbeitungsproduktivität steigert und sich gleichzeitig der Maschinen- und Energieaufwand reduziert. Weiter wird so ein Verbleiben von Reststoffen der Trägermatrix reduziert. Dieses begünstigt wiederum die Senkung des allergenen Potentials.

Aufgrund ihrer hohen Härte ist für solche 100 %-Systeme die Hauthaftung problematisch.

Es ist ferner bekannt, derartige Selbstklebemassen nicht nur vollflächig sondern auch rasterpunktförmig aufzubringen, beispielsweise durch Siebdruck (DE-PS 42 37 252), wobei die Klebstoffpünktchen auch unterschiedlich groß und/oder unterschiedlich verteilt sein können (EP-PS 353 972), oder durch Tiefdruck von in Längs- und Querrichtung zusammenhängenden Stegen (DE-PS 43 08 649).

Der Vorteil des rasterförmigen Auftrags besteht darin, daß die Klebematerialien bei entsprechend porösem Trägermaterial luft- und wasserdampfdurchlässig sowie in der Regel leicht wieder ablösbar sind.

Ein Nachteil dieser Produkte besteht jedoch in der Tatsache, daß bei zu hoher Flächendeckung der an sich undurchlässigen Klebeschicht die Luft- und Wasserdampfdurchlässigkeit sich entsprechend verringert, sowie der Klebemassenverbrauch steigt und bei geringer Flächendeckung der Klebeschicht die Klebeeigenschaften leiden, d.h., das Produkt löst sich, insbesondere bei schweren, textilen Trägermaterialien, zu leicht vom Untergrund.

Im Stande der Technik sind bereits zahlreiche Ausführungsformen wirkstoffhaltiger Pflaster beschrieben worden, die zum Teil nach dem Reservoirprinzip arbeiten, bei welchem der Wirkstoff beispielsweise über eine Membran abgegeben wird, teilweise auch mit einem Matrixsystem oder mit komplizierterem mehrschichtigen Aufbau.

Es ist ferner bekannt, daß die Klebemasse des Pflasters als den Wirkstoff enthaltende Matrix eingesetzt werden kann. Neben aus Lösung aufgetragenen Selbstklebemassen wurden auch schon Heißschmelzklebemassen dafür vorgeschlagen, zum Beispiel in der EP-A 663 431, EP-A 452 034, EP-A 305 757, DE-OS 43 10 012, DE-OS 42 22 334 und DE-C 42 24 325. Als Wirkstoffe wurden hierbei, wenn diese benannt wurden, systemisch wirkende aufgeführt. Eine Verwendung des erfindungsgemäßen Trägers ist insbesondere in DE-C 42 24 325 nicht beschrieben.

Ein Wirkstoffenthaltende klebemasse wird dotierte klebemasse bezeichnet.

Beispielhaft für wirkstoffhaltige Pflaster seien die durchblutungsfördernden Wirkstoffpflaster genannt, die zu der Gruppe der lokal wirksamen therapeutischen Systeme gehören. Die Anwendung solcher Pflaster ist angezeigt zur Behandlung von rheumatischen Beschwerden, Ischias, Hexenschuß, Nackensteiflgkeit, Schulter-Arm-Schmerzen sowie Muskelverspannungen und -zerrungen, Muskelkater oder Muskel-, Gelenk- und Nervenschmerzen im Bereich des Bewegungsapparates.

Capsaicin, Belladonna und Nonivamid sind bekannte Wirkstoffe solcher lokal, durchblutungsfördernd wirkender Pflaster. Aufgrund ihrer Anwendung am Bewegungsapparat müssen sie in der Regel stark kleben. Üblicherweise werden die Pflaster vollflächig mit einer Harz-Kautschuk-Klebemasse beschichtet, welche den Wirkstoff enthält.

Derartige Pflaster, welche meist größer flächig aufgebracht werden müssen, können jedoch nach dem Ablösen bei empfindlichen Patienten fallweise zu mechanische Hautimtationen führen.

Der geschilderte Nachteil trifft ebenso auch auf wirkstoffhaltige Pflaster zu, die andere als die genannten Stoffe beinhalten.

So beschreibt die WO 94/02123 ein Wirkstoffpflaster auf Basis von Haftschmelzklebemassen, welche niedrigschmelzende und/oder leicht flüchtige Wirkstoffe in einer Konzentration von 2,5 Gew.-% bis 25 Gew.-% enthält. Eine Verwendung des erfindungsgemäßen Trägers ist nicht beschrieben. EP 0 663 431 A2, EP 0 443 759 A3, EP 0 452 034 A2 und US 5,371,128 beschreiben Verwendungen von druckempfindlichen Schmelzhaftklebern auf Silikonbasis mit diversen Additiven und in differenzierten Aufbauformen. Eine Verwendung des erfindungsgemäßen Trägers ist nicht beschrieben.

DE 43 10 012 A1 beschreibt den Aufbau eines dermalen therapeutischen Systems aus schmelzfähigen Poly(meth)acrylat-Mischungen.

Insbesondere scheint die Freigabe von mehreren Wirkstoffen aus Polymersystemen, welche nur eine Polymertype schwierig. Systeme mit mehreren Polymertypen sind jedoch kritisch in ihrer Verträglichkeit. Eine Verwendung des erfindungsgemäßen Trägers ist nicht beschrieben.

DE 43 16 751 C1 beschreibt ein Mehrkammersystem zur Darreichung von Wirkstoffen.

EP 0 439 180 beschreibt ein Wirkstoffpflaster zur Darreichung von Tulobuterol.

EP 0 305 757 beschreibt ein Wirkstoffpflaster zur Darreichung von Nicotin.

EP 0 305 758 beschreibt ein Wirkstoffpflaster zur Darreichung von Nitroglycerin.

EP 0 305 756 beschreibt eine Klebemasse von Stoffen und ihrer Herstellung und Verwendung. Eine Verwendung des erfindungsgemäßen Trägers ist nicht beschrieben.

DE 37 43 945 beschreibt eine Klebemasse zur Abgabe von Stoffen sowie das Herstellverfahren. Bei der beschriebenen Haftschmelzklebemasse auf Basis von SIS handelt es sich um keine selbstklebende Vorrichtung. Die dort angegebenen Verarbeitungsbereiche liegen deutlich unter denen von Heißschmelzklebemassen und würden für solche beschriebenen Systeme keine ausreichende Verankerung der Klebemasse bieten. Eine Verwendung des erfindungsgemäßen Trägers ist nicht beschrieben.

WO 96/22083 zeigt einen Polyisobutylenkleber für transdermale Zwecke, welcher einen Klebrigmacher mit hohem Glasübergangspunkt hat. Eine Verwendung des erfindungsgemäßen Trägers ist nicht beschrieben.

JP 07-196505 beschreibt eine Darreichung von Inkomethacin in Schmelzhaftklebern. Es wird hier ein Polyethylenschaum als Trägermaterial verwendet. Eine Verwendung des erfindungsgemäßen Trägers ist nicht beschrieben.

Aufgabe der Erfindung war es, ein Trägermaterial auf Vliesbasis zur Verfügung zu stellen, welches mit einer dotierten Klebemasse beschichtet worden ist und für medizinische Anforderungen geeignet ist und das darüber hinaus die aus dem Stand der Technik bekannten Nachteile nicht aufweist.

Gelöst wird diese Aufgabe durch ein Vlies, wie es im Hauptanspruch niedergelegt ist. Eine alternative Ausführungsform ist Gegenstand des nebengeordneten Anspruches 2. Die Untersprüche umfassen vorteilhafte Varianten des Erfindungsgegenstands.

Demgemäß wird ein mittels Nähfäden übernähtes Vlies als Trägermaterial für medizinische Zwecke verwendet, wobei die Anzahl der Nähte auf dem Vlies vorteilhafterweise mindestens 3/cm, bevorzugt 5/cm bis 50/cm, beträgt. Die Höchstzugkraft des Trägermaterials beläuft sich auf mindestens 10 N/cm, bevorzugt 20 bis 450 N/cm, besonders bevorzugt 30 bis 250 N/cm, und das Trägermaterial ist zumindest einseitig mit einer dotierten Klebemasse partiell oder vollflächig beschichtet.

Die Nähfäden weisen vorzugsweise eine Wasseraufnahme von weniger als 30 % auf, besonders bevorzugt weniger als 20%. Die Wasseraufnahme kann durch die Luftfeuchtigkeit regeneriert werden.

Als Materialien für die Nähfäden können demzufolge vorteilhafterweise polymere Fasern aus Polypropylen, Polyester, Polyamid, Aramid oder Polyethylen sowie auch mineralische Fasern wie Glasfasern oder Kohlenstoffasern eingesetzt werden. Weiter können auch Zwirne oder Mischzwirne, insbesondere Siromischzwirne, eingesetzt werden. Für spezielle Anwendungen können auch Mischfaserfäden, -garne oder -zwirne eingesetzt werden. Weiterhin können die Nähfäden wenigstens teilweise gefärbt sein, um das Trägermaterial optisch ansprechender zu gestalten.

Für spezielle Anwendungen kann der Nähfaden auch elastisch sein. Hieraus regeneriert sich dann ein elastischer Basisträger mit einer Dehnung bis zu 250 % bei einer Belastung von 10 N/cm. Beispielsweise sei hier als ein Polyamidfaden (Lycra ®, Fa. DuPont) erwähnt.

Ein derartiges Trägermaterial erzeugt bei einer Dehnung von 20 % bis 70 % eine Kompressionskraft von 0,2 N/cm bis 10 N/cm und wird in der Kompressionstechnik eingesetzt.

Bei der alternativen Ausführungsform des Erfindungsgegenstands wird ein Vlies als Trägermaterial für medizinische Zwecke eingesetzt. Das Vlies ist dabei durch die Bildung von Nähten, die von Maschen aus den Fasern des Vlieses gebildet werden, verstärkt, wobei vorteilhafterweise die Anzahl der Nähte auf dem Vlies mindestens 3/cm, bevorzugt 5/cm bis 50/cm, beträgt. Die Höchstzugkraft des Trägermaterials beträgt mindestens 10 N/cm, bevorzugt 20 bis 450 N/cm, besonders bevorzugt 50 bis 250 N/cm, und das Trägermaterial ist zumindest einseitig mit einer dotierten Klebemasse partiell oder vollflächig beschichtet.

Die Trägermaterialien basieren auf bekannten Vliesen, die mechanisch verfestigt sind, und zwar durch das Übernähen mit separaten Fäden oder durch das Vermaschen.

Im ersten Falle ergeben sich die Vlies-Faden-Nähgewirke. Zur Herstellung dieser wird ein Faservlies vorgelegt, das beispielsweise quergetäfelt sein kann und mittels separater Fäden in Fransen- oder Trikotlegung übernäht wird.

Diese Vliese sind unter dem Namen "Maliwatt" (von der Firma Malimo) oder Arachne bekannt.

Bei der zweiten Art der Verfestigung wird ebenfalls vorzugsweise ein quergetäfeltes Vlies vorgelegt. Während des Verfestigungsvorganges ziehen Nadeln aus dem Vlies selbst Fasern heraus und formen sie zu Maschen, wobei in Fransenlegung Nähte entstehen. Dieses Vlies-Nähgewirke ist unter dem Namen "Malivlies", ebenfalls von der Firma Malimo, verbreitet.

Eine Übersicht über die verschiedenen Arten der mechanisch verfestigten Faservliesstoffe ist dem Artikel "Kaschierung von Autopolsterstoffen mit Faservliesen" von G. Schmidt, Melliand Textilberichte 6/1992, Seiten 479 bis 486, zu entnehmen.

Vorteilhafterweise weisen die Vliese Längsnähte auf, wobei die Orientierung der Nähfäden entsprechend der Beanspruchung des Trägermaterials im Gebrauch ausgerichtet sein sollte.

Als Ausgangsmaterialien für den Vliesstoff können generell alle organischen und anorganischen Faserstoffe auf natürlicher und synthetischer Basis verwendet werden. Beispielhaft seien Viskose, Baumwolle, Seide, Polypropylen, Polyester, Polyurethan, Polyamid, Aramid oder Polyethylen sowie auch mineralische Fasem wie Glasfasern oder Kohlenstoffasern angeführt. Die vorliegende Erfindung ist aber nicht auf die genannten Materialien beschränkt, sondern es können eine Vielzahl weiterer Fasern zur Vliesbildung eingesetzt werden.

Die zur Bildung des Vlieses eingesetzten Fasern weisen vorzugsweise ein Wasserrückhaltevermögen auf von mehr als 0,5 %, bevorzugt zwischen 2 bis 70 %, besonders bevorzugt zwischen 3 und 50 %.

In einer bevorzugten Ausführungsform weist das Trägermaterial eine Höchstzugkraftdehnung unter 40 %, bevorzugt unter 15 %, besonders bevorzugt unter 10 %, auf.

Dies wird für ein mittels Nähfäden übemähtes Vlies zum einen durch die Verwendung eines Nähfadenmaterials mit hohem Elastizitätsmodul, zum anderen durch die Verwendung eines Nähstiches, der eine möglichst gestreckte Nähfadenlage gewährleistet, erreicht.

Vorteilhafte Materialkombinationen sind zum Beispiel Nähfäden aus hochfesten Polymerfasem wie Polyamid, Polyester, hochverstrecktem Polyethylen oder Mineralfasern wie Glas und vorgelegten Vliesmaterialien wie Baumwolle oder Zellwolle.

Für ein Vlies, bei dem die Bildung von Nähten dadurch erfolgt, daß die Maschen aus den Fasern des Vlieses gebildet werden, ist das Material des vorgelegten Vlieses entsprechend auszuwählen, für den Nähstich gilt Entsprechendes.

Weiterhin als vorteilhaft hat sich herausgestellt, wenn das Trägermaterial ein Flächengewicht bis zu 500 g/m$^2$, bevorzugt 10 bis 350 g/m$^2$, aufweisen soll.

In einer weiteren vorteilhaften Ausführungsform ist das Trägermaterial von Hand senkrecht zur Orientierung der Nähte und/oder in Richtung der Nähte reißbar. Dieses findet dann Anwendung, wenn das erfindungsgemäße Trägermaterial auf sich selbst zur Rolle gewickelt wird.

Bei einer anderen Ausführungsform ist diese Reißbarkeit nicht notwendig. Hier kann es sich um vorgestanzte Pflasterstoffe handeln.

Des weiteren kann das Trägermaterial mit einem Faden oder mehreren Fäden aus Monofil, Multifil, Stapelfasergarn oder Spinnfasergarn und/oder mit orientierten hochfesten Fasern verstärkt sein, wobei die Fäden und/oder Fasern insbesondere eine Festigkeit von mindestens 40 cN/tex aufweisen.

Weiter können auch Zwirne oder Mischzwirne, insbesondere Siromischzwime, eingesetzt werden. Für spezielle Anwendung können auch Mischfaserfäden, -garne oder- zwirne eingesetzt werden. Hierbei kann es sich zum Beispiel um Umwindegarne oder spezielle Stapelfaserumwindegarne handeln.

Vorteilhaft ist hier, daß durch die Kombination von verschiedenen Fasertypen besondere oder spezielle Eigenschaften schon in dem Verstärkungsfaden erreicht werden können. Beispielhaft sind hier die Kombinationen von Polyester oder Polyamid mit Baum- oder Zellwolle.

Die Verstärkungsfasern oder -fäden können dabei aus organischen oder anorganischen Materialien bestehen, so beispielsweise und bevorzugt aus Glas, Kohlenstoff, Polyester oder speziellen Polyamiden bestehen, weiterhin können die Verstärkungsfasem wenigstens teilweise gefärbt sein, um das Trägermaterial optisch ansprechender zu gestalten. Auf diese Art ist es problemlos möglich, die verstärkten Träger optisch zu differenzieren. Hierzu bieten sich insbesondere gefärbte Glas- oder Polymerfäden an.

Bei einer vorteilhaften Ausführungsvariante zeigt das Trägermaterial durch den Zusatz von hochfesten Fasern oder Fäden mit einer Höchstzugkraft über 40 cN/tex eine Höchstzugkraft über 50 N/cm und eine Höchstzugkraftdehnung unter 25% bei einem Flächengewicht von unter 140 g/m$^2$.

Die Anzahl der an- oder eingebrachten Fäden beziehungsweise hochfesten Fasern hängt in erster Linie vom jeweils vorgesehenen Verwendungszweck und der angestrebten Höchstzugkraft sowie Höchstzugkraftdehnung des Trägermaterials, seiner eigenen Beschaffenheit und der jeweiligen Festigkeit der Fasern und Fäden selbst ab und kann deshalb in relativ weiten Grenzen variieren.

Vorteilhafte Materialkombinationen sind zum Beispiel Verstärkungsfäden oder -fasern aus hochfesten Polymerfasern wie Polyamid, Polyester, hochverstrecktem Polyethylen oder Mineralfasern wie Glas und vorgelegten Vliesmaterialien wie Baumwolle oder Zellwolle.

Weiter werden die Verstärkungen vorzugsweise gezielt entsprechend der Beanspruchungsrichtung des Trägermaterials eingefügt, d.h. in Längsrichtung. Sie können jedoch auch, wenn dies zweckdienlicher ist, zusätzlich oder nur in Quer- oder Schrägrichtung oder beispielsweise kurven-, spiral- oder zick-zackförmig oder regellos verlaufen.

In einer weiteren vorteilhaften Ausführungsform ist das Trägermaterial von Hand senkrecht zur Orientierung der Verstärkung und/oder in Richtung der Verstärkung reißbar.

Mit höherer Festigkeit des Vliesmaterials sowie steigenden Anteils an Verstärkungsfasern hält der Träger einer größeren Beanspruchung und Belastung stand. Auch sehr stark verstärkte Trägermaterialien sind in der Lage, große Mengen an Feuchtigkeit aufzunehmen oder durchzulassen, und üben somit eine angenehme Anwenderempfindung aus, wobei die Verstärkungen keine beziehungsweise nur eine geringe Menge an Feuchtigkeit aufnehmen, wodurch sich ihre Eigenschaften nicht ändern.

in der Klebemasse können vorzugsweise unterschiedliche wirkende Stoffe enthalten sein, wobei weiter vorzugsweise die Mengenkonzentrationen des oder der Wirkstoffe in der Klebmasse zwischen 0,01 bis ca. 60 Gew.-%, vorzugsweise 0,1 bis 20 Gew.-%, liegen.

Unter der Bezeichnung "Wirkstoffe" im Zusammenhang mit der vorliegenden Erfindung werden chemische Elemente, organische und anorganische Verbindungen, die aus den sie enthaltenden Bestandteilen einer gattungsgemäßen Klebemasse herauswandern können und dadurch einen angestrebten Effekt hervorrufen, verstanden. Unter den Anwendungsgebieten der erfindungsgemäßen Klebemasse ist die Human- und Tiermedizin von besonderer Bedeutung, wobei hier eine Ausgestaltung der Erfindung in Pflasterform besonders bevorzugt ist.

Typische, über erfindungsgemäß hergestellte Vorrichtungen verabreichbare Stoffe sind hierbei:

Aceclidin, Amfetaminil, Amfetamin, Amylnitrit, Apophedrin, Atebrin, Alpostadil, Azulen, Arecolin, Anethol, Amylenhydrat, Acetylcholin, Acridin, Adenosintriphosphorsäure, L-Äpfelsäure, Alimemazin, Allithiamin, Allyl Isothiocyanat, Aminoethanol, Apyzin, Apiol, Azatadin, Alprenolol, Äthinazon, Benzoylperoxid, Benzylalkohol, Bisabolol, Bisnorephedrin, Butacetoluid, Benactyzin, Campher, Colecalciferol, Chloralhydrat, Clemastin, Chlorobutanol, Capsaicin, Cyclopentamin, Clobutinol, Chamazulen, Dimethocain, Codein, Chloropromazin, Chinin, Chlorthymol, Cyclophosphamid, Cinchocain, Chlorambuzil, Chlorphenesin, Diethylethan, Divinylethan, Dexchlorpheniramin, Dinoproston, Dixyrazin, Ephedrin, Ethosuximid, Enallylpropymal, Emylcamat, Erytroltetranitrat, Emetin, Enfluran, Eucalyptol, Etofenamat, Ethylmorphin, Fentanyl, Fluanison, Guajazulen, Halothan, Hyoscyamin, Histamin, Fencarbamid, Hydroxycain, Hexylresorcin, Isoaminilcitrat. Isosorbiddinitrat, Ibuprofen, Jod, Jodoform, Isoaminil, Lidocain, Lopirin, Levamisol, Methadon, Methyprylon, Methylphenidat, Mephenesin, Methylephedrin, Meclastin, Methopromazin, Mesuximid, Nicethamid, Norpseudoephedrin, Menthol, Methoxyfluran, Methylpentinol, Metixen, Mesoprostol, Oxytetracain, Oxyprenolol, Oxyphenbutazon, Oxychinolin, Pinen, Prolintan, Procyclidin, Piperazin, Pivazid, Phensuximid, Procain, Phenindamin, Promethazin, Pentetrazol, Profenamin, Perazin, Phenol, Pethidin, Pilocarpin, Prenylamin, Phenoxybenzamin, Resochin, Scopolamin, Salicylsäureester, Spartein, Trichlorethylen, Timolol, Trifluperazin, Tetracain, Trimipramin, Tranylcypromin, Trimethadion, Tybamat, Thymol, Thioridazin, Valproinsäure, Verapamil, sowie weitere, dem Fachmann geläufige, über die Haut, eingeschlossen Schleimhäute, aufnehmbare Wirkstoffe. Selbstverständlich ist diese Aufzählung nicht abschließend.

Die Verteilung der Wirkstoffe in der Klebemasse erfolgt vorzugsweise in marktüblichen Homogenisator, wie zum Beispiel Mixer, Kneter, Walzwerke oder Schneckensysteme. Die Zugabe des Wirkstoffs kann in die vollständig hergestellte Klebemasse erfolgen. Beispielhaft kann der Wirkstoff auch in eine Zwischenstufe oder in die Ausgangsmischung eingearbeitet werden.

Als Klebemassen lassen sich vorteilhafterweise Selbstklebemassen einsetzen auf Basis natürlicher und synthetischer Kautschuke und anderer synthetischer Polymere wie Acrylate, Methacrylate, Polyurethane, Polyolefine, Polyvinylderivate, Polyester oder Silikone mit entsprechenden Zusatzstoffen wie Klebharzen, Weichmachern, Stabilisatoren und anderen Hilfsstoffen soweit erforderlich.

Die Klebemasse kann weiterhin Polymere enthalten, welche ein physikalisch oder chemisch vernetztes Gel ausbilden.

Insbesondere thermoplastische Heißschmelzklebemassen weisen vorteilhafte Eigenschaften auf und sind aus produktionstechnischen Gründen begünstigt.

Ihr Erweichungspunkt sollte höher als 50°C liegen, da die Applikationstemperatur bei der Beschichtung in der Regel mindestens 90°C beträgt, bevorzugt zwischen 120°C und 150 °C beziehungsweise 180°C und 220 °C bei speziellen Klebemassen wie Silikonen.

Gegebenenfalls kann eine Nachvernetzung durch energiereiche Strahlung wie UV- oder Elektronenstrahlen angebracht sein.

Bevorzugte Heißschmelzklebemassen auf Basis von Blockcopolymeren zeichnen sich durch ihre vielfältige Variationsmöglichkeiten aus, denn durch die gezielte Absenkung der Glasübergangstemperatur der Selbstklebemasse infolge der Auswahl der Klebrigmacher, der Weichmacher sowie der Polymermolekülgröße und der Molekularverteilung der Einsatzkomponenten wird die notwendige funktionsgerechte Verklebung mit der Haut auch an kritischen Stellen des menschlichen Bewegungsapparates gewährleistet.

Die hohe Scherfestigkeit der Heißschmelzklebemasse wird durch die hohe Kohäsivität des Polymeren erreicht. Die gute Anfaßklebrigkeit ergibt sich durch die eingesetzte Palette an Klebrigmachem und Weichmachern.

Für besonders starkklebende Systeme basiert die Heißschmelzklebemasse bevorzugt auf Blockcopolymeren, insbesondere A-B-, A-B-A-Blockcopolymere oder deren Mischungen. Die harte Phase A ist vornehmlich Polystyrol oder dessen Derivate, und die weiche Phase B enthält Ethylen, Propylen, Butylen, Butadien, Isopren oder deren Mischungen, hierbei besonders bevorzugt Ethylen und Butylen oder deren Mischungen.

Polystyrolblöcke können aber auch in der weichen Phase B enthalten sein, und zwar bis zu 20 Gew.-%. Der gesamte Styrolanteil sollte aber stets niedriger als 35 Gew.-% liegen. Bevorzugt werden Styrolanteile zwischen 5% und 30%, da ein niedrigerer Styrolanteil die Klebemasse anschmiegsamer macht.

Insbesondere die gezielte Abmischung von Di-Block- und Tri-Blockcopolymeren ist vorteilhaft, wobei ein Anteil an Di-Blockcopolymeren von kleiner 80 Gew.-% bevorzugt wird.

In einer vorteilhaften Ausführung weist die Heißschmelzklebemasse die nachfolgend angegebene Zusammensetzung auf:

| 10 Gew.-% bis 90 Gew.-% | Blockcopolymere, |
|---|---|
| 5 Gew.-% bis 80 Gew.-% | Klebrigmacher wie Öle, Wachse, Harze und/oder deren Mischungen, bevorzugt Mischungen aus Harzen und Ölen, |
| weniger als 60 Gew.-% | Weichmacher, |
| weniger als 15 Gew.-% | Additive, |
| weniger als 5 Gew.-% | Stabilisatoren. |
| weniger als 60 Gew.-% | Wirkstoff |

Die als Klebrigmacher dienenden aliphatischen oder aromatischen Öle, Wachse und Harze sind bevorzugt Kohlenwasserstofföle, -wachse und -harze, wobei sich die Öle, wie Paraffinkohlenwasserstofföle, oder die Wachse, wie Paraffinkohlenwasserstoffwachse, durch ihre Konsistenz günstig auf die Hautverklebung auswirken. Als Weichmacher finden mittel- oder langkettige Fettsäuren und/oder deren Ester Verwendung. Diese Zusätze dienen dabei der Einstellung der Klebeeigenschaften und der Stabilität. Gegebenenfalls kommen weitere Stabilisatoren und andere Hilfsstoffe zum Einsatz.

Ein Füllen der Klebemasse mit mineralischen oder organischen Füllstoffen, Fasern, Mikrohohl- oder -vollkugeln ist möglich.

Die Selbstklebemasse weist einen Erweichungspunkt auf oberhalb von 70 °C, bevorzugt 80 °C bis 140 °C.

Die Heißschmelzselbstklebemassen sind vorzugsweise so eingestellt, daß sie bei einer Frequenz von 0,1 rad/s eine dynamisch-komplexe Glasübergangstemperatur von weniger als 15 °C, bevorzugt von 3 °C bis -30 °C, ganz besonders bevorzugt von -3 °C bis-25 °C, aufweisen.

Insbesondere an medizinische Trägermaterialien werden hohe Anforderungen bezüglich der Klebeeigenschaften gestellt. Für eine ideale Anwendung sollte die Heißschmelzklebemasse eine hohe Anfaßklebrigkeit besitzen. Die funktionsangepaßte Klebkraft auf der Haut und auf der Trägerrückseite sollte vorhanden sein. Weiterhin ist, damit es zu keinem Verrutschen kommt, eine hohe Scherfestigkeit der Heißschmelzklebemasse notwendig.

Durch die gezielte Absenkung der Glasübergangstemperatur der Klebemasse infolge der Auswahl der Klebrigmacher, der Weichmacher sowie der Polymermolekülgröße und

der Molekularverteilung der Einsatzkomponenten wird die notwendige funktionsgerechte Verklebung mit der Haut und der Trägerrückseite erreicht.

Die hohe Scherfestigkeit der hier eingesetzten Klebemasse wird durch die hohe Kohäsivität des Blockcopolymeren erreicht. Die gute Anfaßklebrigkeit ergibt sich durch die eingesetzte Palette an Klebrigmachem und Weichmachem.

Die Produkteigenschaften wie Anfaßklebrigkeit, Glasübergangstemperatur und Scherstabilität lassen sich mit Hilfe einer dynamisch-mechanischen Frequenzmessung gut quantifizieren. Hierbei wird ein schubspannungsgesteuertes Rheometer verwendet.

Die Ergebnisse dieser Meßmethode geben Auskunft über die physikalischen Eigenschaften eines Stoffes durch die Berücksichtigung des viskoelastischen Anteils. Hierbei wird bei einer vorgegebenen Temperatur die Selbstklebemasse zwischen zwei planparallelen Platten mit variablen Frequenzen und geringer Verformung (linear viskoelastischer Bereich) in Schwingungen versetzt. Über eine Aufnahmesteuerung wird computerunterstützt der Quotient ($Q = \tan \delta$) zwischen dem Verlustmodul (G'' viskoser Anteil) und dem Speichermodul (G' elastischer Anteil) ermittelt.

$$Q = \tan \delta = G''/G'$$

Für das subjektive Empfinden der Anfaßklebrigkeit (Tack) wird eine hohe Frequenz gewählt sowie für die Scherfestigkeit eine niedrige Frequenz.

Eine hoher Zahlenwert bedeutet eine bessere Anfaßklebrigkeit und eine schlechtere Scherstabilität.

Die Glasübergangstemperatur ist die Temperatur, bei der amorphe oder teilkristalline Polymere vom flüssigen oder gummielastischen Zustand in den hartelastischen oder glasigen Zustand übergehen oder umgekehrt (Römpp Chemie-Lexikon, 9. Aufl., Band 2, Seite 1587, Georg Thieme Verlag Stuttgart - New York, 1990). Er entspricht dem Maximum der Temperaturfunktion bei vorgegebener Frequenz.

Besonders für medizinische Anwendungen ist ein relativ niedriger Glasübergangspunkt notwendig.

| Bezeichnung | $T_G$ niedrige Frequenz | Anschmiegsamkeit niedrige Frequenz/RT | Anfaßklebrigkeit hohe Frequenz/RT |
|---|---|---|---|
| Heißschmelzklebemasse A | $-10 \pm 2°C$ | $\tan \delta = 0{,}35 \pm 0{,}05$ | $\tan \delta = 1{,}70 \pm 0{,}10$ |
| Heißschmelzklebemasse B | $-9 \pm 2°C$ | $\tan \delta = 0{,}22 \pm 0{,}03$ | $\tan \delta = 1{,}00 \pm 0{,}03$ |

Bevorzugt werden erfindungsgemäß Selbstklebemassen, bei denen das Verhältnis des viskosen Anteils zum elastischen Anteil bei einer Frequenz von 100 rad/s bei 25 °C größer 0,7 ist, oder Selbstklebemassen, bei denen das Verhältnis des viskosen Anteils zum elastischen Anteil bei einer Frequenz von 0,1 rad/s bei 25°C kleiner 0,4 ist, bevorzugt zwischen 0,35 und 0,02, ganz besonders bevorzugt zwischen 0,3 und 0,1.

Vorteilhaft insbesondere für die Verwendung bei medizinischen Produkten ist weiterhin, wenn die Klebemasse partiell auf dem Trägermaterial aufgetragen ist, beispielsweise durch Rasterdruck, Siebdruck, Thermoflexodruck oder Tiefdruck, denn im Vollstrich klebend ausgerüstete Trägermaterialien können unter ungünstigen Voraussetzungen bei der Applikation mechanische Hautirritationen hervorrufen und sind in der Regel nicht Luft- und wasserdampfdurchlässig.

Bevorzugt wird der Auftrag in Form von polygeometrischen Kalotten und ganz besonders von solchen Kalotten, bei denen das Verhältnis Durchmesser zu Höhe kleiner 5:1 ist. Weiterhin ist auch der Aufdruck anderer Formen und Muster auf dem Trägermaterial möglich, so beispielsweise ein Druckbild in Form alphanumerischer Zeichenkombinationen oder Muster wie Gitter, Streifen und Zickzacklinien.
Die Klebemasse kann gleichmäßig auf dem Trägermaterial verteilt sein, sie kann aber auch funktionsgerecht für das Produkt über die Fläche unterschiedlich stark oder dicht aufgetragen sein.

Das Prinzip des Thermosiebdrucks besteht in der Verwendung einer rotierenden beheizten, nahtlosen, trommelförmigen perforierten Rundschablone, die über eine Düse mit der bevorzugten Heißschmelzselbstklebemasse beschickt wird. Eine speziell geformte Düsenlippe (Rund- oder Vierkantrakel) preßt die über einen Kanal eingespeiste Heißschmelzklebemasse durch die Perforation der Schablonenwand auf die vorbeigeführte Trägerbahn. Diese wird mit einer Geschwindigkeit, die der Umgangsgeschwindigkeit der rotierenden Siebtrommel entspricht, mittels einer Gegendruckwalze gegen den Außenmantel der beheizten Siebtrommel geführt.

Die Ausbildung der kleinen Klebstoffkalotten geschieht dabei nach folgendem Mechanismus:

Der Düsenrakeldruck fördert die Heißschmelzklebemasse durch die Siebperforation an das Trägermaterial. Die Größe der ausgebildeten Kalotten wird durch den Durchmesser des Siebloches vorgegeben. Entsprechend der Transportgeschwindigkeit der Trägerbahn (Rotationsgeschwindigkeit der Siebtrommel) wird das Sieb vom Träger abgehoben. Bedingt durch die hohe Adhäsion der Klebemasse und die innere Kohäsion des Hotmelts wird von der auf dem Träger bereits haftenden Basis der Kalotten der in den Löchern begrenzte Vorrat an Heißschmelzklebemasse konturenscharf abgezogen beziehungsweise durch den Rakeldruck auf den Träger gefördert.
Nach Beendigung dieses Transportes formt sich, abhängig von der Rheologie der Heißschmelzklebemasse, über der vorgegebenen Basisfläche die mehr oder weniger stark gekrümmte Oberfläche der Kalotte. Das Verhältnis Höhe zur Basis der Kalotte hängt vom Verhältnis Lochdurchmesser zur Wandstärke der Siebtrommel und den physikalischen Eigenschaften (Fließverhalten, Oberflächenspannung und Benetzungswinkel auf dem Trägermaterial) der Selbstklebemasse ab.

Bei der Siebschablone im Thermosiebdruck kann das Steg/Loch-Verhältnis kleiner 3:1 sein, bevorzugt kleiner oder gleich 1:1, insbesondere gleich 1:3.

Der beschriebene Bildungsmechanismus der Kalotten erfordert bevorzugt saugfähige oder zumindest von Heißschmelzklebemasse benetzbare Trägermaterialien. Nichtbenetzende Trägeroberflächen müssen durch chemische oder physikalische Verfahren vorbehandelt werden. Dies kann durch zusätzliche Maßnahmen wie zum Beispiel Coronaentladung oder Beschichtung mit die Benetzung verbessernden Stoffen geschehen.

Mit dem aufgezeigten Druckverfahren kann die Größe und Form der Kalotten definiert festgelegt werden. Die für die Anwendung relevanten Klebkraftwerte, die die Qualität der erzeugten Produkte bestimmen, liegen bei sachgerechter Beschichtung in sehr engen Toleranzen. Der Basisdurchmesser der Kalotten kann von 10 µm bis 5000 µm gewählt werden, die Höhe der Kalotten von 20 µm bis ca. 2000 µm, bevorzugt 50 um bis 1000 µm, wobei der Bereich kleiner Durchmesser für glatte Träger, der mit größerem Durchmesser und größerer Kalottenhöhe für rauhe oder stark porige Trägermaterialien vorgesehen ist.
Die Positionierung der Kalotten auf dem Träger wird durch die in weiten Grenzen variierbare Geometrie des Auftragswerkes, zum Beispiel Gravur- oder Siebgeometrie, definiert festgelegt. Mit Hilfe der aufgezeigten Parameter kann über einstellbare Größen das gewünschte Eigenschaftsprofil der Beschichtung, abgestimmt auf die verschiedenen Trägermaterialien und Anwendungen, sehr genau eingestellt werden.

Das Trägermaterial wird bevorzugt mit einer Geschwindigkeit von größer 2 m/min, bevorzugt 20 bis 200 m/min, beschichtet, wobei die Beschichtungstemperatur größer als die Erweichungstemperatur zu wählen ist.

Die Selbstklebemasse kann mit einem Flächengewicht von größer 15 g/m$^2$, bevorzugt zwischen 90 g/m$^2$ und 500 g/m$^2$, ganz besonders bevorzugt zwischen 130 g/m$^2$ und 500 g/m$^2$, auf dem Trägermaterial aufgetragen sein.

Der prozentuale Anteil, der mit der Selbstklebemasse beschichteten Fläche sollte mindestens 20 % betragen und kann bis zu ungefähr 95 % reichen, für spezielle Produkte bevorzugt 40 % bis 60 % sowie 70 % bis 95 %. Dieses kann gegebenenfalls durch Mehrfachapplikation erreicht werden, wobei gegebenenfalls auch Selbstklebemassen mit unterschiedlichen Eigenschaften eingesetzt werden können.

Die Kombination der bevorzugten Heißschmelzklebemasse und der bevorzugten partiellen Beschichtung sichert auf der einen Seite eine sichere Verklebung des medizinischen Produktes auf der Haut, auf der anderen Seite sind zumindest visuell erkennbare allergische oder mechanische Hautirritationen ausgeschlossen, auch bei einer Anwendung, die sich über mehrere Tage erstreckt.

Die Epilation entsprechender Körperregionen und der Massetransfer auf die Haut sind aufgrund der hohen Kohäsivität des Klebers vernachlässigbar, weil der Kleber nicht an Haut und Haar verankert, vielmehr ist die Verankerung der Klebemasse auf dem Trägermaterial mit bis zu 12 N/cm (Probenbreite) für medizinische Anwendungen gut.

Durch die ausgeformten Sollbruchstellen in der Beschichtung werden Hautlagen beim Ablösen nicht mehr mit- oder gegeneinander verschoben. Das Nichtverschieben der Hautlagen und die geringere Epilation führen zu einem bisher nicht gekannten Grad der Schmerzfreiheit bei solchen stark klebenden Systemen. Weiter unterstützt die individuelle biomechanische Klebkraftsteuerung, welche eine nachweisliche Absenkung der Klebkraft dieser Pflaster aufweist, die Ablösbarkeit. Das applizierte Trägermaterial zeigt gute propriorezeptive Wirkungen.

Je nach Trägermaterial und dessen Temperaturempfindlichkeit kann die Heißschmelzklebemasse direkt aufgetragen sein oder zuerst auf einen Hilfsträger aufgebracht und dann auf den endgültigen Träger transferiert werden. Auch ein nachträgliches Kalandern des beschichteten Produktes und/oder eine Vorbehandlung des Trägers, wie Coronabestrahlung, zur besseren Verankerung der Klebeschicht kann vorteilhaft sein.

Weiterhin kann eine Behandlung der Heißschmelzklebemasse mit einer Elektronenstrahl-Nachvernetzung oder einer UV-Bestrahlung zu einer Verbesserung der gewünschten Eigenschaften führen.

In einer weiteren vorteilhaften Ausführungsform werden die Selbstklebemassen geschäumt, bevor sie auf das Trägermaterial aufgetragen werden.

Die Selbstklebemassen werden dabei bevorzugt mit inerten Gasen wie Stickstoff, Kohlendioxid, Edelgasen, Kohlenwasserstoffen oder Luft oder deren Gemischen geschäumt. In manchen Fällen hat sich ein Aufschäumen zusätzlich durch thermische Zersetzung gasentwickelnder Substanzen wie Azo-, Carbonat- und Hydrazid-Verbindungen als geeignet erwiesen.

Der Schäumungsgrad, d.h. der Gasanteil, sollte mindestens etwa 5 Vol.-% betragen und

kann bis zu etwa 85 Vol.-% reichen. In der Praxis haben sich Werte von 10 Vol.-% bis 75 Vol.-%, bevorzugt 50 Vol.-%, gut bewährt. Wird bei relativ hohen Temperaturen von ungefähr 100°C und vergleichsweise hohem Innendruck gearbeitet, entstehen sehr offenporige Klebstoffschaumschichten, die besonders gut luft- und wasserdampfdurchlässig sind.

Die vorteilhaften Eigenschaften der geschäumten Selbstklebebeschichtungen wie geringer Klebstoffverbrauch, hohe Anfaßklebrigkeit und gute Anschmiegsamkeit auch an unebenen Flächen durch die Elastizität und Plastizität sowie der Initialtack lassen ganz besonders sich auf dem Gebiet der medizinischen Produkte optimal nutzen.

Durch den Einsatz von atmungsaktiven Beschichtungen in Verbindung mit elastischen ebenfalls atmungsaktiven Trägermaterialien ergibt sich ein vom Anwender subjektiv angenehmer empfundener Tragekomfort.

Ein besonders geeignetes Verfahren zur Herstellung der geschäumten Selbstklebemasse arbeitet nach dem Schaum-Mix-System. Hierbei wird die thermoplastische Selbstklebemasse unter hohem Druck bei einer Temperatur über dem Erweichungspunkt (ungefähr 120 °C) mit den vorgesehenen Gasen wie zum Beispiel Stickstoff, Luft oder Kohlendioxid in unterschiedlichen Volumenanteilen (etwa 10 Vol.-% bis 80 Vol.-%) in einem Stator/Rotorsystem umgesetzt.

Während der Gasvordruck größer 100 bar ist, betragen die Mischdrucke Gas/Thermoplast im System 40 bis 100 bar, bevorzugt 40 bis 70 bar. Der so hergestellte Haftklebeschaum kann anschließend über eine Leitung in das Auftragswerk gelangen. Bei dem Auftragswerk finden handelsübliche Düsen, Extruder- oder Kammersysteme Verwendung.

Durch die Schäumung der Selbstklebemasse und die dadurch entstandenen offenen Poren in der Masse sind bei Verwendung eines an sich porösen Trägers die mit der Klebemasse beschichteten Produkte gut wasserdampf- und luftdurchlässig. Die benötigte Klebemassenmenge wird erheblich reduziert ohne Beeinträchtigung der Klebeeigenschaften. Die Klebemassen weisen eine überraschend hohe Anfaßklebrigkeit (tack) auf, da pro Gramm Masse mehr Volumen und damit Klebeoberfläche zum Benetzen des zu beklebenden Untergrundes zur Verfügung steht und die Plastizität der Klebemassen durch die Schaumstruktur erhöht ist. Auch die Verankerung auf dem Trägermaterial wird dadurch verbessert. Außerdem verleiht die geschäumte Klebebeschichtung, wie bereits oben erwähnt, den Produkten ein weiches und anschmiegsames Anfühlen.

Durch das Schäumen wird zudem die Viskosität der Klebemassen in der Regel gesenkt. Hierdurch wird die Schmelzenergie erniedrigt, und es können auch thermoinstabile Trägermaterialien direkt beschichtet werden.

Das mit der Klebemasse beschichtete Trägermaterial kann eine Luftdurchlässigkeit von größer 1 cm$^3$/(cm$^2$*s), bevorzugt größer 15 cm$^3$/(cm$^2$*s), ganz besonders bevorzugt größer 70 cm$^3$/(cm$^2$*s), aufweisen, des weiteren eine Wasserdampfdurchlässigkeit von größer 500 g/(m$^2$*24h), bevorzugt größer 1000 g/(m$^2$*24h), ganz besonders bevorzugt größer 2000 g/(m$^2$*24h).

Darüber hinaus kann das Trägermaterial auch mit weiteren Ausrüstungen oder Behandlungen versehen sein. Dazu zählen zum Beispiel Corona-, Flamm- oder Plasmavorbehandlungen, um die Verankerung der Selbstklebemasse auf dem Basisvlies zu erhöhen. Auch das Kalandern des Trägermaterials oder des noch nicht mit Selbsthaftkleber ausgerüsteten Vlieses zum Zwecke der weiteren Verfestigung beziehungsweise der Verbesserung der Verankerung des Selbsthaftklebers ist eine vorteilhafte Behandlung.

Weiterhin kann das Trägermaterial auf der Seite, die der mit Selbstklebemasse beschichteten Seite gegenüberliegt, mit einer wasserabweisenden Schicht oder Imprägnierung ausgerüstet sein, die eine schnelle Durchnässung bei Kontakt mit Wasser oder Schweiß verhindert. Neben den bekannten Imprägnierungen kann dies auch durch das Aufnähen einer Folie, vorteilhafterweise einer wasserdampfdurchlässigen Folie, direkt beim Verfestigen des Vlieses durch Nähfäden erfolgen.

Das Trägermaterial kann darüber hinaus mit einer die Klebkraft der Selbstklebemasse vermindernden Releaseschicht oder -imprägnierung und/oder Lackierung ausgerüstet sein. Auch hierbei kann neben den bekannten Releasematerialien eine Folie, vorteilhafterweise eine wasserdampfdurchlässige Folie, direkt beim Verfestigen des Vlieses aufgenäht werden.

Vorteilhaft hat sich auch das Laminieren des Vliesträgers mit zumindest einer zusätzlichen Schicht aus Schäumen oder Folien herausgestellt, da sich hierdurch eine Kombination von Eigenschaften besonderer Art ergibt. Ein Schaum hat eine wesentlich höhere Dämpfungseigenschaft auf als ein nicht laminierter Träger. Folien können zum Beispiel zum Versiegeln der Oberfläche verwendet werden.

Des weiteren kann das Trägermaterial mit metallischen Substanzen bedampft werden.

Schließlich kann das Trägermaterial nach dem Beschichtungsvorgang mit einem klebstoffabweisenden Trägermaterial, wie silikonisiertes Papier, eingedeckt oder mit einer Wundauflage oder einer Polsterung versehen werden.

Besonders vorteilhaft ist, daß das Trägermaterial sterilisierbar, bevorzugt γ-(gamma) sterilisierbar, ist. So sind besonders geeignet für eine nachträgliche Sterilisation Heißschmelzklebemassen auf Blockcopolymerbasis, welche keine Doppelbindungen enthalten. Dieses gilt ins besondere für Styrol-Butylen-Ethylen-Styrol-Blockcopolymerisate oder Styrol-Butylen-Styrol-Blockcopolymerisate. Es treten hierbei keine für die Anwendung signifikanten Änderungen in den Klebeeigenschaften auf.

Das erfindungsgemäße Trägermaterial weist eine Klebkraft auf der Trägerrückseite von mindestens 0,5 N/cm auf, besonders eine Klebkraft zwischen 2,5 N/cm und 5 N/cm. Auf anderen Untergründen können höhere Klebkräfte erreicht werden.

Vorteilhaft zeigte sich, daß ein so klebend beschichtetes, verstärktes Trägermaterial beim Durchnässen, wie es zum Beispiel bei wassersportlichen Aktivitäten unvermeidlich ist, eine gegenüber den handelsüblichen Trägermaterialien verbesserte Stabilität innehat. Die relative Zunahme der Höchstzugkraft-Dehnung erfindungsgemäßer selbstklebend ausgerüsteter Trägermaterialien ist nach dem Durchnässen nur halb so groß wie bei handelsüblichen selbstklebend ausgerüsteten Trägermaterialien.

Hierdurch werden die erfindungsgemäßen Trägermaterialien, die ja somit im wesentlichen unelastisch sind, für spezielle medizinische Zwecke nutzbar, und weiter können Trägermaterialien, deren Einsatz bisher an mangelnder Festigkeit und/oder zu hoher Dehnung gescheitert ist, eingesetzt werden.

Bevorzugt können Trägermaterialien auf Basis von Gewebe, Gewirke, Vliese oder Verbundprodukte verwendet werden, sofern sie ansonsten die Anforderungen an den medizinischen Einsatz erfüllen.

**Beispiel 1**

Im folgenden sei beispielhaft ein bevorzugtes Trägermaterial beschrieben, ohne damit die Erfindung unnötig einschränken zu wollen.

Als Trägermaterial wurde ein Vliesstoff auf Basis von Viskose verwendet. Der Vliesstoff wurde mit einen Polyesterfaden übernäht, wobei die Nähfadenzahl 22/cm Probenbreite betrug. Die Wasseraufnahme des Polyesterfadens betrug 0,3 %. Das Trägermaterial wurde kalandert und imprägniert.

Das so hergestellte Trägermaterial wies in Längsrichtung eine Höchstzugkraft von 50 N/cm und eine Höchstzugkraft-Dehnung von 28% auf. Das Flächengewicht betrug 120 g/cm$^2$. Ein vollständiges Durchnässen war bei dem Trägermaterial aufgrund der Auswahl der Verstärkungsmaterialien nicht möglich. Das Trägermaterial hat unbelastet eine Dicke von 1,0 mm was einen guten Polsterungseffekt hervorruft.

Das Trägermaterial wies eine Luftdurchlässigkeit von 100 cm$^3$/(cm$^2$*s) und Wasserdampfdurchlässigkeit von größer 2500 g/(m$^2$*24h) auf und ließ sich von Hand in Querrichtung und Längsrichtung ein- und durchreißen.

Die Heißschmelzklebemasse wurde im Thermosiebdruck auf den Träger appliziert. Die Heißschmelzklebemasse setzte sich wie folgt zusammen:

- ein A-B/A-B-A Blockcopolymer, welches aus harten und weichen Segmenten besteht, mit einem Verhältnis der A-B-A zur A-B von 2:1 und einem Styrolgehalt im Polymer von 13 Mol.-%; der Anteil an der Klebemasse beträgt 40 Gew.-% (Kraton G),
- ein Paraffinkohlenwasserstoffwachs mit einem Anteil an der Klebmasse von 51 Gew.-%,
- Kohlenwasserstoffharze mit einem Anteil von 7,5 Gew.-% (Super Resin HC 140),
- ein Alterungsschutzmittel mit einem Anteil von weniger als 0,5 Gew.-% (Irganox).
- ein hyperämisierender Wirkstoff (Nonanonyl Vanillylamid); Anteil von 1%.

Die eingesetzten Komponenten wurden in einem Thermomischer bei 125 °C homogenisiert. Die Glasübergang betrug nach oben genannter Methode -9 °C.

Die direkte Beschichtung erfolgte mit 50 m/min bei einer Temperatur von 120 °C. Das Trägermaterial wurde mit 120 g/m$^2$ partiell beschichtet, wobei eine 14 Meshsieb-Schablone mit einer Siebstärke von 300 μm verwendet wurde.

Die nach diesem Verfahren hergestellte Binde zeigte ein reversibles Ablösen von der Haut sowie eine gute Luft- und Wasserdampfdurchlässigkeit. Es wurden keine Hautirritationen und eine vernachlässigbar geringe Epilation nach dem Abnehmen der Binde beobachtet.

Das so hergestellte Trägermaterial zeigt eine gute Freisetzung des Wirkstoffes.

**Beispiel 2**

Als Trägermaterial wurde ein Vliesstoff auf Basis von Viskose verwendet. Der Vliesstoff wurde mit einen Polyamidfaden übernäht, wobei die Nähfadenzahl 7/cm Probenbreite betrug. Das Trägermaterial wurde kalandert und imprägniert.

Das so hergestellte Trägermaterial wies in Längsrichtung eine Höchstzugkraft von 12 N/cm und eine Höchstzugkraft-Dehnung von 28% auf. Das Flächengewicht betrug 140 g/cm$^2$. Ein vollständiges Durchnässen war bei dem Trägermaterial aufgrund der Auswahl der Verstärkungsmaterialien nicht möglich. Das Trägermaterial hat unbelastet eine Dicke von 1,2 mm was einen guten Polsterungseffekt hervorruft.

Das Trägermaterial wies eine Luftdurchlässigkeit von 80 cm$^3$/(cm$^2$*s) und Wasserdampfdurchlässigkeit von größer 2500 g/(m$^2$*24h) auf und ließ sich von Hand in Querrichtung und Längsrichtung ein- und durchreißen.

Diese Schmelzhaftktebemasse setzte sich wie folgt zusammen:

- ein SEPS Blockcopolymer, welches aus harten und weichen Segmenten besteht und einem Styrolgehalt im Polymer von 13 Mol.-%; der Anteil an der Klebemasse beträgt 15 Gew.-% (Kuraray Co.)
- ein Paraffinkohlenwasserstoffharz mit einem Anteil an der Klebmasse von 44 Gew.-%
- ein Alterungsschutzmittel mit einem Anteil von weniger als 1,0 Gew.-% (Irganox 1010 Ciba)
- Salicylsäure mit einem Anteil von 40%

Die eingesetzten Kleberkomponenten wurden in einem Thermomischer bei 185°C 3,5 homogenisiert. Der Wirkstoff wurde in der Abkühlphase bei 120°C zugegeben und weitere 130 min im Mischer homogenisiert.

Der Erweichungspunkt dieser Klebmasse betrug ca. 85°C (DIN 52011), und die Klebmasse zeigte eine Viskosität von 2100 mPas bei 150 °C (DIN 53018, Brookfield DV II. Sp. 21). Die Glasübergang betrug nach o.g. Methode - 10 °C. Die Selbstklebemasse wurde mit einer Düse vollflächig auf den Träger appliziert. Die direkte Beschichtung erfolgte mit 50 m/min bei einer Temperatur von 120 °C. Das Trägermaterial wurde mit 170 g/m$^2$ beschichtet.

Das so hergestellte Pflastermaterial zeigt eine vergleichbar gute Freisetzung des Wirkstoffs.

**Patentansprüche**

1. Trägemiaterial für medizinische Zwecke, **dadurch gekennzeichnet, daß** das Trägermaterial ein mittels Nähfäden übemähtes Vlies mit einer Höchstzugkraft von mindestens 10 N/cm ist und daß das Trägermaterial zumindest einseitig mit einer dotierten Klebemasse partiell oder vollflächig beschichtet ist.

2. Trägermaterial für medizinische Zwecke, **dadurch gekennzeichnet, daß** das Trägermaterial ein Vlies ist, das durch die Bildung von Nähten, die von Maschen aus den Fasern des Vlieses gebildet werden, verstärkt ist. wobei die Höchstzugkraft des Trägermaterials mindestens 10 N/cm beträgt und daß das Trägermaterial zumindest einseitig mit einer dotierten Selbstklebemasse partiell oder vollflächig beschichtet ist.

3.  Trägermaterial für medizinische Zwecke nach Anspruch 1 oder 2, **dadurch gekennzeichnet, daß** die Anzahl der Nähte, insbesondere Längsnähte, auf dem Vlies mindestens 3/cm, bevorzugt 5 bis 50/cm, beträgt.

4.  Trägermaterial für medizinische Zwecke nach Anspruch 4, **dadurch gekennzeichnet, daß** das Trägermaterial bei einer Dehnung von 20 % bis 70 % eine Kompressionskraft von 0,2 N/cm bis 10 N/cm erzeugt.

5.  Trägermaterial für medizinische Zwecke nach Anspruch 1 oder 2, **dadurch gekennzeichnet, daß** das Trägermaterial ein Flächengewicht bis zu als 500 g/m$^2$, bevorzugt 10 bis 350 g/m$^2$, aufweist.

6.  Trägermaterial für medizinische Zwecke nach Anspruch 1 oder 2, **dadurch gekennzeichnet, daß** das Trägermaterial mit einem Faden oder mehreren Fäden aus Monofil, Multifil, Stapelfasergam oder Spinnfasergam und/oder mit orientierten hochfesten Fasern verstärkt ist, wobei die Fäden und/oder Fasern insbesondere eine Festigkeit von mindestens 40 cN/tex aufweisen.

7.  Trägermaterial für medizinische Zwecke nach Anspruch 1 oder 2, **dadurch gekennzeichnet, daß** das Trägermaterial von Hand senkrecht zur Orientierung der Nähte und/oder in Richtung der Nähte und/oder senkrecht zur Orientierung der Verstärkung und/oder in Richtung der Verstärkung reißbar ist.

8.  Trägermaterial für medizinische Zwecke gemäß mindestens einem der vorangegangenen Anspruch, **dadurch gekennzeichnet, daß** die Klebemasse den oder die freisetzbaren Wirkstoffe in einer Menge von 0,01 bis 60 Gew.-%, bevorzugt von 0,1 bis 20 Gew.-%, enthält.

9.  Trägermaterial für medizinische Zwecke gemäß mindestens einem der vorangegangenen Anspruch, **dadurch gekennzeichnet, daß** die Klebemasse eine Heißschmelzklebemasse ist.

10. Trägermaterial für medizinische Zwecke gemäß mindestens einem der vorangegangenen Anspruch, **dadurch gekennzeichnet, daß** die Klebemasse bei einer Frequenz von 0,1 rad/s eine dynamisch-komplexe Glasübergangstemperatur von weniger als 15°C, bevorzugt von 3°C bis -30°C, ganz besonders bevorzugt von -3°C bis-25 °C, aufweist.

11. Trägermaterial für medizinische Zwecke gemäß mindestens einem der vorangegangenen Anspruch, **dadurch gekennzeichnet, daß** die Klebemasse geschäumt wurde.

12. Trägermaterial für medizinische Zwecke gemäß mindestens einem der vorangegangenen Anspruch, **dadurch gekennzeichnet , daß** die Klebemasse partiell auf dem Trägermaterial aufgetragen, insbesondere durch Rasterdruck, Thermosiebdruck, Thermoflexodruck oder Tiefdruck.

13. Trägermaterial für medizinische Zwecke gemäß mindestens einem der vorangegangenen Anspruch, **dadurch gekennzeichnet, daß** die Klebemasse aufgesprüht ist.

14. Trägermaterial für medizinische Zwecke gemäß mindestens einem der vorangegangenen Anspruch, **dadurch gekennzeichnet, daß** die Klebemasse in Form von polygeometrischen Kalotten auf das Trägermaterial gebracht wird.

15. Trägermaterial für medizinische Zwecke gemäß mindestens einem der vorangegangenen Anspruch, **dadurch gekennzeichnet, daß** die Klebemasse mit einem Flächengewicht von größer 15 g/m$^2$, bevorzugt zwischen 90 g/m$^2$ und 500 g/m$^2$, ganz besonders bevorzugt zwischen 130 g/m$^2$ und 500 g/m$^2$, auf dem Trägermaterial aufgetragen ist.

16. Trägermaterial für medizinische Zwecke gemäß mindestens einem der vorangegangenen Anspruch, **dadurch gekennzeichnet, daß** die Klebemasse sterilisierbar, bevorzugt γ (gamma)-sterilisierbar, ist.

17. Trägermaterial für medizinische Zwecke nach einem oder mehreren der vorhergehenden Ansprüche, **dadurch gekennzeichnet, daß** das Trägermaterial auf der Seite, die der mit der Selbstklebemasse beschichteten Seite gegenüberliegt, mit einer wasserabweisenden Schicht, Imprägnierung, Releaseschicht und/oder Lackierung ausgerüstet ist.

18. Trägermaterial für medizinische Zwecke nach einem oder mehreren der vorhergehenden Ansprüche, **dadurch gekennzeichnet, daß** auf das Trägermaterial zumindest eine zusätzliche Schicht aus Folie, Schäumen oder Vliesen aufgebracht ist.

19. Trägermaterial für medizinische Zwecke nach einem oder mehreren der vorhergehenden Ansprüchen, **dadurch gekennzeichnet, daß** das Trägermaterial mit metallischen Substanzen bedampft worden ist.

20. Trägermaterial für medizinische Zwecke nach einem oder mehreren der vorhergehenden Ansprüche, **dadurch gekennzeichnet, daß** das beschichtete Trägermaterial nach der Applikation der Selbstklebemasse eingedeckt oder mit einer Wundauflage oder Polsterung versehen wird.

**Claims**

1. Backing material for medical purposes, **characterized in that** the backing material is a nonwoven overstitched by means of yarns and having an ultimate tensile stress strength of at least 10 N/cm and **in that** the backing material is coated partially or over its full area on at least one side with a doped adhesive composition.

2. Backing material for medical purposes, **characterized in that** the backing material is a nonwoven which is reinforced by the formation of stitches formed by loops from the fibres of the web, the ultimate tensile stress strength of the backing material being at least 10 N/cm, and **in that** the backing material is coated partially or over its full area on at least one side with a doped self-adhesive composition.

3. Backing material for medical purposes according to Claim 1 or 2, **characterized in that** the number of stitches on the web, in particular longitudinal stitches, is at least 3/cm, preferably from 5 to 50/cm.

4. Backing material for medical purposes according to Claim 4, **characterized in that** the backing material generates a compression force of from 0.2 N/cm to 10 N/cm at an elongation of from 20% to 70%.

5. Backing material for medical purposes according to Claim 1 or 2, **characterized in that** the backing material has a basis weight of up to 500 g/m$^2$, preferably from 10 to 350 g/m$^2$.

6. Backing material for medical purposes according to Claim 1 or 2, **characterized in that** the backing material is reinforced with one or more monofil, multifil, staple fibre or spun fibre yarns and/or with oriented high-strength fibres, the yarns and/or fibres having in particular a strength of at least 40 cN/tex.

7. Backing material for medical purposes according to Claim 1 or 2, **characterized in that** the backing material can be torn by hand perpendicular to the orientation of the stitches and/or in the direction of the stitches and/or perpendicular to the orientation of the reinforcement and/or in the direction of the reinforcement.

8. Backing material for medical purposes according to at least one of the preceding claims, **characterized in that** the adhesive composition comprises the releasable active substance or substances in an amount of from 0.01 to 60% by weight, preferably from 0.1 to 20% by weight.

9. Backing material for medical purposes according to at least one of the preceding claims, **characterized in that** the adhesive composition is a hot-melt adhesive composition.

10. Backing material for medical purposes according to at least one of the preceding claims, **characterized in that** the adhesive composition has a dynamic-complex glass transition temperature at a frequency of 0.1 rad/s of less than 15°C, preferably from 3°C to -30°C, with very particular preference from -3°C to -25°C.

11. Backing material for medical purposes according to at least one of the preceding claims, **characterized in that** the adhesive composition was foamed.

12. Backing material for medical purposes according to at least one of the preceding claims, **characterized in that** the adhesive composition is applied partially to the backing material, especially by halftone printing, thermal screen printing, thermal flexographic printing or intaglio printing.

13. Backing material for medical purposes according to at least one of the preceding claims, **characterized in that** the adhesive composition has been sprayed on.

14. Backing material for medical purposes according to at least one of the preceding claims, **characterized in that** the adhesive composition is applied in the form of polygeometric domes to the backing material.

15. Backing material for medical purposes according to at least one of the preceding claims, **characterized in that** the adhesive composition is applied to the backing material with a weight per unit area of greater than 15 g/m$^2$, preferably between 90 g/m$^2$ and 500 g/m$^2$, with very particular preference between 130 g/m$^2$ and 500 g/m$^2$.

16. Backing material for medical purposes according to at least one of the preceding claims, **characterized in that** the adhesive composition can be sterilized, preferably with γ (gamma) radiation.

17. Backing material for medical purposes according to one or more of the preceding claims, **characterized in that** on the side opposite that coated with the self-adhesive composition, the backing material is finished with a water-repellent layer, impregnation, release layer and/or coating.

18. Backing material for medical purposes according to one or more of the preceding claims, **characterized in that** at least one additional layer comprising sheets, foams or nonwovens is applied on the backing material.

19. Backing material for medical purposes according to one or more of the preceding claims, **characterized in that** the backing material is coated with metallic substances by vapour deposition.

20. Backing material for medical purposes according to one or more of the preceding claims, **characterized in that** the coated backing material is covered after application of the self-adhesive composition or is provided with a wound pad or with padding.


**Revendications**

1. Matériau support pour applications médicales, **caractérisé en ce que** ledit matériau support consiste en un voile surpiqué de fils à coudre et d'une force de traction maximale d'au moins 10 N/cm et **en ce que** ce matériau support est revêtu partiellement ou complètement, au moins sur une face, d'une colle dopée.

2. Matériau support pour applications médicales, **caractérisé en ce que** ce matériau support consiste en un voile renforcé par des coutures formées à partir de mailles des fibres du voile, la force de traction maximale étant d'au moins 10 N/cm et **en ce que** ce matériau support est revêtu partiellement ou complètement, au moins sur une face, d'une colle dopée.

3. Matériau support pour applications médicales selon la revendication 1 ou 2, **caractérisé en ce que** le nombre de coutures, en particulier les coutures longitudinales est d'au moins 3/cm sur le voile, de préférence de 5 à 50/cm.

4. Matériau support pour applications médicales selon la revendication 4, **caractérisé en ce que** ce matériau support offre, pour une extension de 20% à 70%, une force de compression de 0,2 N/cm à 10 N/cm.

5. Matériau support pour applications médicales selon la revendication 1 ou 2, **caractérisé en ce que** ce matériau support présente un grammage jusqu'à 500 g/m$^2$, de préférence de 10 à 350 g/m$^2$.

6. Matériau support pour applications médicales selon la revendication 1 ou 2, **caractérisé en ce que** ce matériau support est renforcé d'un ou de plusieurs fil(s) en monofil, multifil, fibre textile coupée ou filée et/ou de fibres orientées à haute résistance, les fils et/ou fibres présentant en particulier une solidité d'au moins 40 cN/tex.

7. Matériau support pour applications médicales selon la revendication 1 ou 2, **caractérisé en ce que** le matériau support est déchirable manuellement, perpendiculairement à la direction des coutures et/ou dans la direction des coutures et/ou perpendiculairement à la direction du renforcement et/ou dans la direction du renforcement.

8. Matériau support pour applications médicales selon au moins l'une quelconque des revendications précédentes, **caractérisé en ce que** le ou les composé(s) volatil(s) de la colle représentent de 0,01 à 60% en poids, de préférence de 0,1 à 20% en poids.

9. Matériau support pour applications médicales selon au moins l'une quelconque des revendications précédentes, **caractérisé en ce que** la colle est une colle thermofusible.

10. Matériau support pour applications médicales selon au moins l'une quelconque des revendications précédentes, **caractérisé en ce que** la colle présente, à une fréquence de 0,1 rad/s, une température de transition vitreuse dynamico-complexe de moins de 15°C, de préférence de 3°C à -30°C, de manière particulièrement préférée de -3°C à -25°C.

11. Matériau support pour applications médicales selon au moins l'une quelconque des revendications précédentes, **caractérisé en ce que** la colle a été moussée.

12. Matériau support pour applications médicales selon au moins l'une quelconque des revendications précédentes, **caractérisé en ce que** la colle est appliquée partiellement sur le matériau support, en particulier par points, thermosérigraphie, thermoflexographie ou rotogravure.

13. Matériau support pour applications médicales selon au moins l'une quelconque des revendications précédentes, **caractérisé en ce que** la colle est appliquée par projection.

14. Matériau support pour applications médicales selon au moins l'une quelconque des revendications précédentes, **caractérisé en ce que** la colle est appliquée sur le matériau support sous la forme de calottes polygéométriques.

15. Matériau support pour applications médicales selon au moins l'une quelconque des revendications précédentes, **caractérisé en ce que** la colle est appliquée sur le matériau support en un grammage supérieur à 15 $g/m^2$, de préférence entre 90 $g/m^2$ et 500 $g/m^2$, de manière particulièrement préférée entre 130 $g/m^2$ et 500 $g/m^2$.

16. Matériau support pour applications médicales selon au moins l'une quelconque des revendications précédentes, **caractérisé en ce que** la colle est stérilisable, idéalement stérilisable aux rayons y (gamma).

17. Matériau support pour applications médicales selon l'une ou plusieurs des revendications précédentes, **caractérisé en ce que** ce matériau support est pourvu, sur la face opposée à la face revêtue d'autoadhésif, d'une couche hydrofuge, d'une imprégnation, d'une couche anti-adhésive et/ou d'un vernis.

18. Matériau support pour applications médicales selon l'une ou plusieurs des revendications précédentes, **caractérisé en ce que** le matériau support est pourvu d'au moins une couche supplémentaire de film, de mousses ou de non-tissés.

19. Matériau support pour applications médicales selon l'une ou plusieurs des revendications précédentes, **caractérisé en ce que** le matériau support a été métallisé sous vide.

20. Matériau support pour applications médicales selon l'une ou plusieurs des revendications précédentes, **caractérisé en ce que** le matériau support revêtu, après l'application de l'autoadhésif, est doublé ou pourvu d'un pansement ou d'un rembourrage.